# EUROPEAN PATENT SPECIFICATION

(11) **EP 4 426 198 B1**
(45) Date of publication and mention of the grant of the patent: **17.12.2025**
(21) Application number: 22813711.3
(22) Date of filing: 02.11.2022
(51) Int. Cl.: A61B 5/305, A61B 5/00, G01N 27/414

(54) **INTEGRATED ELECTRONIC CIRCUIT WITH OFFSET COMPENSATION FOR AN IMPLANTABLE PROBE**
INTEGRIERTE ELEKTRONISCHE SCHALTUNG MIT OFFSETKOMPENSATION FÜR EINE IMPLANTIERBARE SONDE
CIRCUIT ÉLECTRONIQUE INTÉGRÉ À COMPENSATION DE DÉCALAGE, DESTINÉ À UNE SONDE IMPLANTABLE

(30) Priority: 03.11.2021 IT 202100027956
(43) Date of publication of application: 11.09.2024
(73) Proprietor: Fondazione Istituto Italiano di Tecnologia, 16163 Genova (GE) (IT)
(72) Inventor: BERDONDINI, Luca, 16163 Genova (GE) (IT); ANGOTZI, Gian Nicola, 16163 Genova (GE) (IT); BOI, Fabio, 16163 Genova (GE) (IT); RODRIGUES RIBEIRO, Joao Filipe, 16163 Genova (GE) (IT)
(74) Representative: Studio Torta S.p.A.
(86) International application number: PCT/IB2022/060551
(87) International publication number: WO 2023/079452

(56) References cited:
- IMFELD K ET AL: "Large-Scale, High-Resolution Data Acquisition System for Extracellular Recording of Electrophysiological Activity", IEEE TRANSACTIONS ON BIOMEDICAL ENGINEERING, IEEE, USA, vol. 55, no. 8, 1 August 2008 (2008-08-01), pages 2064 - 2073, XP011247745, ISSN: 0018-9294
- ANGOTZI G N ET AL: "A low-power, low-area modular architecture for high density neural probes", 2015 7TH INTERNATIONAL IEEE/EMBS CONFERENCE ON NEURAL ENGINEERING (NER), IEEE, 22 April 2015 (2015-04-22), pages 521 - 524, XP033166272, DOI: 10.1109/NER.2015.7146674

## Description

### Technical Field

The present invention refers to an integrated electronic circuit with offset compensation for an implantable probe.

### Background

As is well known, so-called implantable multi-electrode array probes that are adapted to record numerous (in the order of thousands) electrophysiological signals generated, for example, by individual neurons are nowadays available.

In particular, active type probes are known, which each include a plurality of pixels, each of which integrates a respective sensing electrode and a corresponding amplification circuitry.

In use, the sensing electrode is contacted with a corresponding portion of tissue (e.g., brain tissue), in order to acquire a corresponding initial electrical signal, which is then amplified by the corresponding amplification circuitry, so as to generate a corresponding output signal.

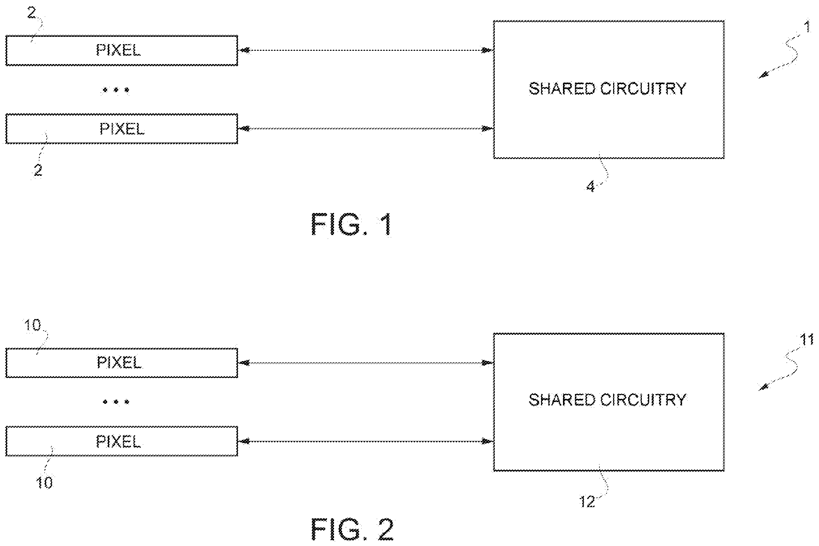

For example, Figure 1 shows a probe 1, which comprises a plurality of pixels 2 and a shared circuitry 4, which is electrically coupled to the pixels 2 (for example, is integrated in the same plate) and is configured so as to allow the parallel acquisition of the output signals generated by the pixels 2, for example by implementing a multiplexing mechanism over time, that is, so as to query each pixel 2 in a corresponding time interval.

In order to have a high density of pixels 2 per unit area, each pixel 2, and therefore also the corresponding amplification circuitry, should occupy a reduced area. For this reason, it is known to use the so-called CMOS technology to create pixels 2, this technology also allowing to reduce consumptions.

However, the Applicant has observed how, in use, a corresponding electrode-electrolyte interface is formed at each sensing electrode and which is subject to high direct voltage variations, i.e. it exhibits a DC offset that can be particularly high (up to hundreds of millivolts). The causes of this DC offset may include, for example, the differences in composition of the biological tissue being measured.

In order to reduce the impact of the aforementioned DC offset on the output signal, it is for example possible to implement an AC coupling between the amplification circuitries and the sensing electrodes, however this solution requires the formation of capacitors with high capacitance, resulting in high area occupancy. This solution is therefore unsuitable for high channel density recording systems, i.e. for recording systems having a high number of pixels per unit area.

The article "Large scale, high-resolution data acquisition system for extracellular recording of electrophysiological activity", by Imfeld K. et al., IEEE TRANSACTIONS ON BIOMEDICAL ENGINEERING, vol.5, no. 8, pp. 2064-2073 and the article "A low-power, low-area modular architecture for high-density neural probes", by Angotzi G.N. and Berdondini L., 7th Annual International IEEE EMBS Conference on Neural Engineering, Montpellier, France, 20-22 April 2015 describe electronic circuits in which an electrode is polarised by means of a pair of P-MOS transistors connected in parallel, which together deliver a current that polarises a further P-MOS transistor, whose gate terminal is connected to the electrode.

### Summary

Aim of the present invention is therefore to provide an integrated electronic circuit that allows to solve at least in part the drawbacks of the prior art.

According to the invention, there is provided an integrated electronic circuit as defined in the appended claims.

### Brief Description of the Figures

For a better understanding of the invention, embodiments thereof will now be disclosed, for merely illustrative and non-limiting purposes and with reference to the enclosed drawings, wherein:
- Figure 1 shows a block diagram of a multipixel probe;
- Figure 2 shows a block diagram of a probe module including the present integrated electronic circuit;
- Figure 3 shows a circuit diagram of a portion of the probe module shown in Figure 2;
- Figure 4 schematically shows a section of a portion of a semiconductive plate;
- Figure 5 shows a block diagram of a circuitry;
- Figure 6 shows a circuit diagram of a portion of the probe module;
- Figure 7 shows examples of trends over time of signals;
- Figures 8 and 9 show circuit diagrams of this integrated electronic circuit, under different operating conditions; and
- Figure 10 shows a further circuit diagram of the probe module.

### Description of embodiments

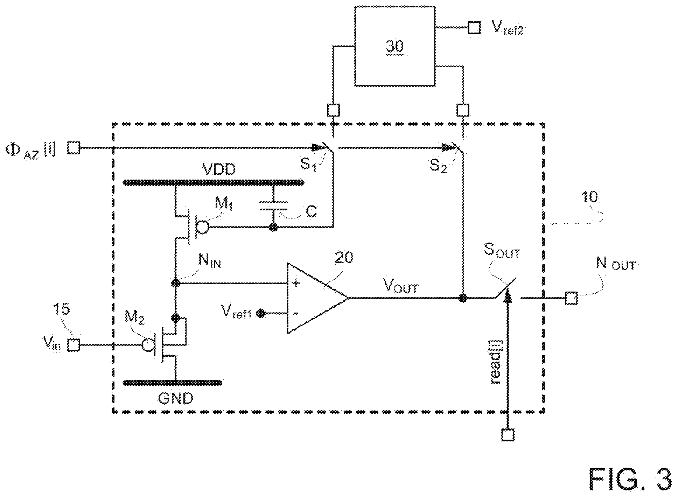

Figure 2 shows a multi-electrode and implantable type probe module 11, which includes a shared circuitry 12 and a plurality of pixels 10 equal to each other and electrically coupled to the shared circuitry 12. For example, the pixels 10 may be thirty-two in number. One of such pixel 10 is shown in Figure 3 and is described below.

In detail, the pixel 10 comprises a supply terminal, which in use is set to a supply voltage VDD for example equal to 1.8V, and a ground (GND). Furthermore, the pixel 10 comprises a first and a second transistor M₁, M₂, which are referred to hereinbelow as the first and second biasing transistors M₁, M₂ respectively.

The first and second biasing transistors M₁, M₂ are P-channel enhancement MOSFET transistors. Furthermore, the source terminal of the first biasing transistor M₁ is connected to the supply voltage VDD; the drain terminal of the first biasing transistor M₁ is connected to the source terminal of the second biasing transistor M₂, so as to form an input node N_{IN}. The drain terminal of the second biasing transistor M₂ is grounded; furthermore, and without any loss of generality, the source terminal of the second biasing transistor M₂ is connected to the body terminal of the second biasing transistor M₂.

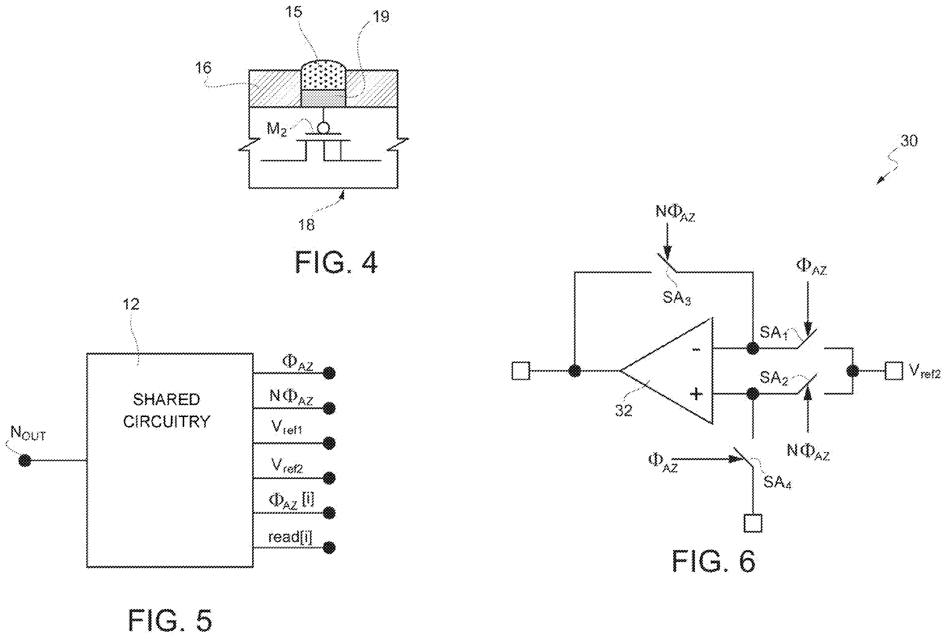

As shown in Figure 4, the gate terminal of the second biasing transistor M₂ is electrically connected to a corresponding electrode 15, which in use is placed in contact with the biological tissue to be analysed (for example, brain tissue), so that on the electrode 15 there is an input voltage Vᵢₙ, which is equal to the sum of a direct voltage V_{DC} and a so-called small signal voltage vᵢₙ.

As shown in Figure 4, the electrode 15 may be formed so that it passes through a dielectric region 16 of a plate ("die") 18 in which the probe module 11 is formed, so that it contacts a metallization 19 (e.g., the upper metallization) of the plate 18, said metallization 19 being electrically connected to the gate terminal of the second biasing transistor M₂.

The pixel 10 further comprises a capacitor C, which has a first and a second terminal, which are respectively connected to the gate terminal of the first biasing transistor M₁ and to the supply voltage VDD.

The pixel 10 further comprises an amplifier 20 (for example, but not limited to, of the transconductance type), the positive input terminal of which is connected to the input node N_{IN}, and the negative input terminal of which is connected to the shared circuitry 12, shown in Figure 5, which sets the negative input terminal to a voltage V_{ref1} (for example equal to 600mV).

The pixel 10 furthermore comprises three switches, which are referred to hereinbelow as the first and second calibration switches S₁, S₂ and as the output switch S_{OUT} respectively. As described in greater detail below, the first and second calibration switches S₁, S₂ are controlled by a signal ϕ_{AZ}[i], while the output switch S_{OUT} is controlled by a signal read[i]. The signal ϕ_{AZ}[i] and the signal read[i] are generated by the shared circuitry 12, as shown in Figure 5 and as described in greater detail below. Furthermore, the output switch S_{OUT} is interposed between the output terminal of the amplifier 20 and an output node Nₒᵤₜ, which in turn is coupled to the shared circuitry 12.

The shared circuitry 12 comprises a feedback amplifier stage 30 (shown in Figure 3), which has a first and a second input terminal; the first input terminal is connected to the shared circuitry 12, so as to be set to a voltage V_{ref2} (for example, equal to 900mV), while the second input terminal is connected to the first terminal of the second calibration switch S₂, the second terminal of which is connected to the output terminal of the amplifier 20.

The feedback amplifier stage 30 has furthermore an output terminal, which is connected to a first terminal of the first calibration switch S₁, the second terminal of which is connected to the first terminal of the capacitor C, and therefore also to the gate terminal of the first biasing transistor M₁.

In greater detail, as shown in Figure 6, the feedback amplifier stage 30 comprises a feedback amplifier 32, formed for example, but not limited to, by a transconductance amplifier, and a first, a second, a third and a fourth additional switch SA₁, SA₂, SA₃, SA₄.

The output terminal of the feedback amplifier 32 forms the output terminal of the feedback amplifier stage 30, therefore it is connected to the first terminal of the first calibration switch S₁.

The first additional switch SA₁ is interposed between the negative input terminal of the feedback amplifier 32 and the first input terminal of the feedback amplifier stage 30, which, as mentioned above, is set to the voltage V_{ref2}.

The second additional switch SA₂ is interposed between the positive input terminal of the feedback amplifier 32 and the first input terminal of the feedback amplifier stage 30.

The third additional switch SA₃ is interposed between the negative input terminal of the feedback amplifier 32 and the output terminal of the feedback amplifier 32.

The fourth additional switch SA₄ is interposed between the positive input terminal of the feedback amplifier 32 and the second input terminal of the feedback amplifier stage 30.

Furthermore, the first and fourth additional switches SA₁, SA₄ are controlled by a signal ϕ_{AZ} generated by the shared circuitry 12. In particular, the first and fourth additional switches SA₁, SA₄ are closed when the signal ϕ_{AZ} is equal to '1' and are open when the signal ϕ_{AZ} is equal to '0'.

The second and the third additional switches SA₂, SA₄ are controlled by a signal Nϕ_{AZ} equal to the logical negation of the signal ϕ_{AZ}. In this way, the second and third additional switches SA₂, SA₃ are open when the signal ϕ_{AZ} is equal to '1' and are closed when the signal ϕ_{AZ} is equal to '0'.

In practice, the first and second biasing transistors M₁, M₂ form a so-called common drain stage, which has a unit gain and receives as input the input voltage Vᵢₙ. Furthermore, as explained below, the common drain stage allows to control the direct voltage present on the positive input terminal of the amplifier 20, which provides a high gain (e.g., higher than 40dB) over a band extending, for example, up to 4kHz. At the output from the amplifier 20 there is an output signal V_{OUT}, which, when read[i]='1', is provided on the output node N_{OUT}, therefore it is provided to the shared circuitry 12, which reads it in a per se known way.

In greater detail, the shared circuitry 12 generates the signals ϕ_{AZ} (and thus also Nϕ_{AZ}) and ϕ_{AZ}[i] so that the pixel 10 operates under different operating conditions.

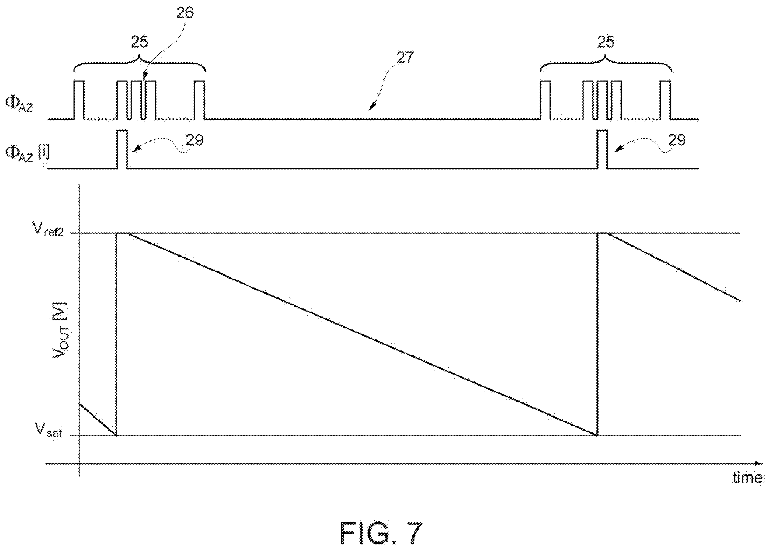

In particular, as shown in Figure 7, the signal ϕ_{AZ} is a signal that provides for an alternation of bursts of pulses (denoted with 25) and of sensing periods (one shown in Figure 7, where it is denoted with 27). Each pulse burst 25 comprises a number of pulses equal to the number of pixels 10.

As far as instead the signal ϕ_{AZ}[i] is concerned, however, it is relative to the i-th pixel 10. Furthermore, considering the i-th pixel 10, the corresponding signal ϕ_{AZ}[i] is generated by the shared circuitry 12 so as to be equal to '1' only when the i-th pixel 10 is selected, otherwise it is equal to '0'; furthermore, the signal ϕ_{AZ} [i] has a single pulse (denoted with 29) for each pulse burst 25 of the signal ϕ_{AZ}, said single pulse 29 being temporally aligned with a corresponding pulse of the pulse burst 25. In addition, when ϕ_{AZ}[i] = '1', ϕ_{AZ}[j]='0' occurs for each j between 1 and 32, but other than i.

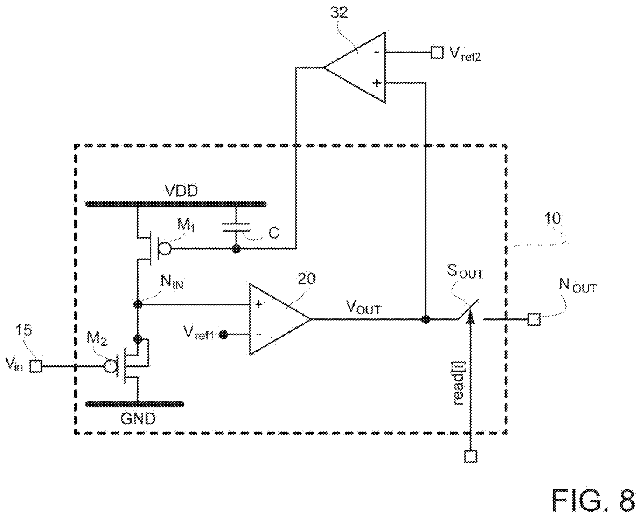

That being said, referring again to the pixel 10 shown in Figure 3, it can operate under a calibration condition, which is given when ϕ_{AZ} [i] = '1', which also entails ϕ_{AZ} = '1'; this condition is shown in Figure 8.

In detail, the first and second calibration switches S₁, S₂ are closed, therefore the feedback amplifier 32 is coupled to the amplifier 20 and forms an autozeroing loop such that a voltage approximately equal to the voltage V_{ref1} is set on the input node N_{IN}. In particular, the capacitor C is subject to a voltage such that the gate terminal of the first biasing transistor M₁ is in turn subject to a voltage such that the first biasing transistor M₁ injects in the input node N_{IN} a biasing current I_{bias}. The biasing current I_{bias} therefore depends on the charge stored in the capacitor C and is precisely such that the input node N_{IN} is set equal to the voltage V_{ref1}.

Furthermore, thanks to the autozeroing loop, on the output terminal of the amplifier 20 there is a voltage approximately equal to the voltage V_{ref2}, as shown again in Figure 7.

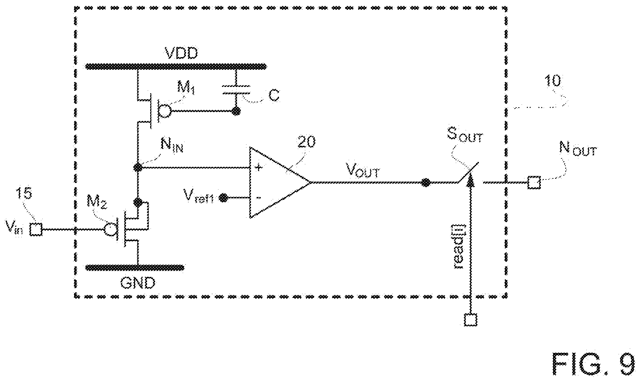

Subsequently, when the signal ϕ_{AZ} [i] returns to '0', the first and second calibration switches S₁, S₁ open and the pixel 10 operates under sensing conditions, shown in Figure 9. In practice, the feedback amplifier stage 30 is decoupled from the amplifier 20 and the autozeroing loop opens.

Ideally, the capacitor C maintains the previously stored charge, therefore the biasing current I_{bias} does not vary and the input node N_{IN} remains at the voltage V_{ref1}; consequently, the biasing of the positive input terminal of the amplifier 20 does not vary. Furthermore, the amplifier 20 operates in open loop and amplifies the input signal V_{IN} present on the electrode 15, allowing a correct sensing thereof. Thus, while the pixel 10 operates under sensing conditions, the shared circuitry 12 can set read[i] = '1', so that the same shared circuitry 12 can read the output signal V_{OUT}, which includes a direct voltage contribution equal to the voltage V_{ref2} and a small signal contribution that is a function of the aforementioned small signal voltage vᵢₙ.

In fact, due to unavoidable losses, the charge present in the capacitor C tends to reduce, causing a reduction of the biasing current I_{bias} and therefore a variation (reduction) of the voltage present on the input node N_{IN}, with consequent reduction of the output signal V_{OUT}, and in particular of the direct voltage component of the latter, as shown in Figure 7. Precisely in order to prevent this reduction from bringing the terminal at the output from the amplifier 20 so close to a limit voltage Vₛₐₜ such that correct amplification is no longer possible, the shared circuitry 12 controls each pixel 10 so as to alternate calibration conditions, in which the voltage V_{ref1} is restored on the input node N_{IN}, at sensing conditions, in which the input signal V_{IN} is read.

Furthermore, during each period of time in which the pixel 10 operates under calibration conditions, the autozeroing loop cancels the impact on the biasing of the amplifier 20 of the DC offset affecting the electrode 15, that is, it compensates for the DC offset. In this regard, thanks to the fact that the biasing current I_{bias} is injected in the input node N_{IN} by a single transistor (in particular, the first biasing transistor M₁), the interval of the DC offset values that can be compensated for (with the same voltage V_{ref1}) is maximized, since the biasing current I_{bias} can be reduced up to a minimum value Iₘᵢₙ which depends on the sizing of the first and second biasing transistors M₁, M₂. In this regard, the direct voltage on the input node N_{IN} is equal to the sum between the direct voltage V_{DC} of the input voltage Vᵢₙ and a direct voltage V_{DCAZ} set by the autozeroing loop; in the presence of high values of the direct voltage V_{DC}, the autozeroing loop reduces the direct voltage V_{DCAZ} (to the limit, until it cancels it, if V_{DC}=V_{ref1}) tending to turn off the first biasing transistor M₁, so as to reduce the biasing current I_{bias}.

Still with reference to the biasing current I_{bias}, the current flowing in the second biasing transistor M₂ is precisely equal to the biasing current I_{bias}, since no current flows in the positive input terminal of the amplifier 20.

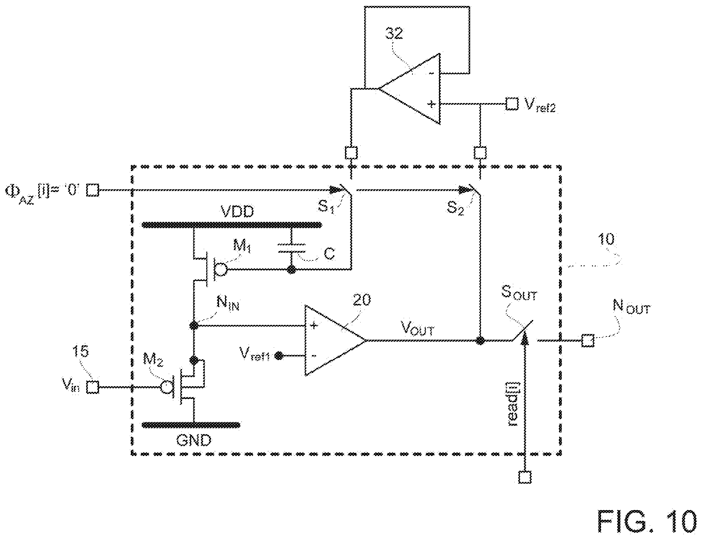

With regard to the signal ϕ_{AZ}, when it is equal to '0', it happens that the second and third additional switches SA₂, SA₃ are closed, while the first and fourth additional switches SA₁, SA₄ are open. Consequently, as shown in Figure 10, the feedback amplifier 32 operates as a voltage buffer and transfer the voltage V_{ref2} to its own output terminal, and thus also to the first terminal of the first calibration switch S₁ of each pixel 10. In this way, the charge losses suffered by the capacitors C of the pixels 10 through the respective first calibration switches S₁ during the sensing periods 27 are reduced, because the voltage at the ends of the first calibration switches S₁ is controlled. The small charge losses that might occur are also reduced, considering a pulse burst 25 of the signal ϕ_{AZ}, during each time interval between two successive pulses of the burst (one shown in Figure 7, where it is denoted with 26).

According to a variant, the voltages V_{ref1} generated on the negative input terminals of the amplifiers 20 of the pixels 10 may vary from pixel to pixel. In other words, the shared circuitry 12 applies, on each negative input terminal of the amplifiers 20 of the pixels 10, a corresponding voltage V_{ref1}, with the following benefits.

In general, the autozeroing loop is capable of compensating for DC offsets in the interval V_{ref1}±ΔV, where ΔV is proportional to the biasing current I_{bias}, which however is subject to an upper limit depending on the dimensions of the first and second biasing transistors M₁, M₂, as well as on the stability conditions of the autozeroing loop. Therefore, high values of the voltage V_{ref1} are better suited to compensate for high values of the DC offset; on the contrary, in case the DC offset has a reduced value, the compensation is preferably obtained by adopting a reduced voltage V_{ref1}.

That being said, since electrodes 15 of different pixels 10 may be subject to different DC offsets, in particular in the event that the probe module 11 includes a very large array of electrodes 15, the possibility of setting, for different pixels 10, different values of the respective voltages V_{ref1} (which are set on the negative input terminals of the respective amplifiers 20) allows to optimize, for each pixel 10, the compensation for the DC offset it experiences.

The advantages that the present integrated electronic circuit allows obtaining emerge clearly from the previous description. In particular, during the calibration step, the autozeroing loop including the feedback amplifier 32 allows to control the biasing current I_{bias} that flows in the common drain stage and therefore allows to adjust the direct voltage present on the positive input terminal of the amplifier 20, compensating for the DC offset present in the voltage Vᵢₙ. Furthermore, since the biasing current I_{bias} comes from a single transistor (in this case, the first biasing transistor M₁), it is possible to compensate for high DC offsets, with the same voltage V_{ref1}.

Furthermore, the Applicant has observed that the autozeroing loop is stable, despite the wide interval of values that can be assumed by the biasing current I_{bias}.

It is finally clear that modifications and variants can be made to this integrated electronic circuit, without departing from the scope of the present invention, as defined by the enclosed claims.

For example, the capacitor C may be made in MIM ("metal-insulator-metal") technology or by using the parasitic capacitance present on the gate terminal of the first biasing transistor M₁.

For example, the negative and positive input terminals of each amplifier 20 and/or of the feedback amplifier may be reversed with respect to what is described.

Furthermore, the probe module 11 can be used together with other probe modules similar to it, so as to implement a system for recording electrical signals of biological origin with high parallelism.

Finally, the capacitor C can be grounded (GND), instead of to the supply voltage VDD.

## Claims

1. An integrated electronic circuit (10,12) for an implantable probe module (11), comprising a number of pixel circuits (10) each including:
- an electrode (15) configured to contact a biological tissue;
- a biasing stage (M₁,C) comprising a capacitor (C) and a first transistor (M₁) having a respective control terminal and a respective first conduction terminal, which are respectively coupled to the capacitor (C) and to an input node (N_{IN}), the first transistor (M₁) being configured to inject in the input node (N_{IN}) a biasing current (I_{bias}) that depends upon the charge of the capacitor (C);
- a second transistor (M₂), which has a respective control terminal and a respective first conduction terminal, which are respectively coupled to the electrode (15) and to the input node (N_{IN});
- an amplifier (20) having a first input terminal, configured to be set to a corresponding first reference voltage (V_{ref1}), and a second input terminal, which is coupled to the input node (N_{IN});
said integrated electronic circuit (10,12) furthermore comprising:
- a feedback stage (30) electrically controllable so as to be alternatively coupled or decoupled from the biasing stage (M₁,C) and from the amplifier (20) of each pixel circuit (10), said feedback stage (30) being configured to form, when coupled to the biasing stage (M₁,C) and to the amplifier (20) of a pixel circuit (10), an autozeroing loop that charges the corresponding capacitor (C) so that the corresponding biasing current (I_{bias}) is such that on the corresponding input node (N_{IN}) a voltage substantially equal to the corresponding first reference voltage (V_{ref1}) is present;
and wherein the feedback stage (30) has a first input terminal, configured to be set to a second reference voltage (V_{ref2}), a second input terminal and a respective output terminal, said feedback stage (30) furthermore comprising a feedback amplifier (32), the output terminal of which is coupled to the output terminal of the feedback stage (30); and wherein each pixel circuit (10) furthermore comprises a respective first switch (S₁), which is controllable so as to couple/decouple the corresponding capacitor (C) to the output terminal of the feedback stage (30), and a respective second switch (S₂), which is controllable so as to couple/decouple the output terminal of the corresponding amplifier (20) to the second input terminal of the feedback stage (30);
said integrated electronic circuit being **characterized in that** the feedback stage (30) is electrically controllable so as to operate alternatively in a first configuration, in which a first and a second input terminal of the feedback amplifier (32) are respectively coupled to the first and to the second input terminals of the feedback stage (30), and in a second configuration, wherein the first and the second input terminals of the feedback amplifier (32) are respectively coupled to the output terminal of the feedback amplifier (32) and to the first input terminal of the feedback stage (30), in such a way that the feedback amplifier (32) operates as a voltage follower and transfers the second reference voltage (V_{ref2}) to the output terminal of the feedback stage (30).

2. The integrated electronic circuit (10,12) according to claim 1, wherein each pixel circuit (10) is configured in such a way that the respective second transistor (M₂) is traversed by the same biasing current (I_{bias}) injected by the corresponding first transistor (M₁).

3. The integrated electronic circuit (10,12) according to claim 1 or 2, wherein the feedback amplifier (32) is a transconductance amplifier.

4. The integrated electronic circuit (10,12) according to any of the preceding claims, furthermore comprising a control circuitry (12) configured to control the first and second switches (S₁,S₂) of the pixel circuits (10) so as to couple the feedback stage (30) to one pixel circuit (10) at a time, said control circuitry (12) being furthermore configured to control the feedback stage (30) so that, when said feedback stage (30) is coupled to a pixel circuit (10), the feedback stage (30) operates in the first configuration; said control circuitry (12) being furthermore configured to alternate first periods of time (25), in which the pixel circuits (10) are individually coupled, in succession, to the feedback stage (30), with second periods of time (27), wherein the feedback stage (30) is decoupled from the pixel circuits (10) and operates in the second configuration.

5. The integrated electronic circuit (10,12) according to any one of the preceding claims, wherein the second transistor (M₂) of each pixel circuit (10) has a respective second conduction terminal, which is set to a first reference potential (GND); and wherein the first transistor (M₁) of each pixel circuit (10) has a respective second conduction terminal, which is set to a second reference potential (VDD); and wherein the capacitor (C) of each pixel circuit (10) has a respective first terminal, set to the first or the second reference potential (VDD), and a respective second terminal, coupled to the control terminal of the corresponding first transistor (M₁).

6. The integrated electronic circuit (10,12) according to any one of the preceding claims, wherein the first and second transistors (M₁,M₂) of each pixel circuit (10) are P-channel enhancement MOSFET transistors.

7. The integrated electronic circuit (10,12) according to any one of the preceding claims, furthermore comprising a biasing circuitry (12) coupled to the first input terminals of the amplifiers (20) of the pixel circuits (10) and configured to set, on each of said input terminals, a corresponding first reference voltage (V_{ref1}).

8. The integrated electronic circuit (10,12) according to any one of the preceding claims, wherein the amplifier (20) of each pixel circuit (10) is configured to generate an output signal (V_{OUT}) on its own output terminal; said integrated electronic circuit (10,12) furthermore comprising a reading circuit (12) couplable in an electrically controllable manner to the pixel circuits (10), so as to read the respective output signals (V_{OUT}).

9. The integrated electronic circuit (10,12) according to any one of the preceding claims, wherein the amplifier (20) of each pixel circuit (10) is a transconductance amplifier.

## Patentansprüche

1. Integrierte elektronische Schaltung (10, 12) für ein implantierbares Sondenmodul (11), die eine Anzahl von Pixelschaltungen (10) umfasst, die jeweils enthalten:
- eine Elektrode (15), die konfiguriert ist, um ein biologisches Gewebe zu kontaktieren;
- eine Vorspannungsstufe (M₁, C), die einen Kondensator (C) und einen ersten Transistor (M₁) mit einem jeweiligen Steueranschluss und einem jeweiligen ersten Leitungsanschluss umfasst, die jeweils an den Kondensator (C) und an einen Eingangsknoten (N_{IN}) gekoppelt sind, wobei der erste Transistor (M₁) konfiguriert ist, um in den Eingangsknoten (N_{IN}) einen Vorspannungsstrom (I_{bias}) einzuspeisen, der von der Ladung des Kondensators (C) abhängt;
- einen zweiten Transistor (M₂), der einen jeweiligen Steueranschluss und einen jeweiligen ersten Leitungsanschluss aufweist, die jeweils mit der Elektrode (15) und dem Eingangsknoten (N_{IN}) gekoppelt sind;
- einen Verstärker (20) mit einem ersten Eingangsanschluss, der konfiguriert ist, um auf eine entsprechende erste Referenzspannung (V_{ref1}) eingestellt zu werden, und einem zweiten Eingangsanschluss, der mit dem Eingangsknoten (N_{IN}) gekoppelt ist;
wobei die integrierte elektronische Schaltung (10, 12) ferner Folgendes umfasst:
- eine Rückkopplungsstufe (30), die elektrisch steuerbar ist, um alternativ mit/von der Vorspannungsstufe (M₁, C) und mit/von dem Verstärker (20) jeder Pixelschaltung (10) gekoppelt oder entkoppelt zu werden, wobei die Rückkopplungsstufe (30) konfiguriert ist, um, wenn sie mit der Vorspannungsstufe (M₁, C) und dem Verstärker (20) einer Pixelschaltung (10) gekoppelt ist, eine Auto-Nullpunkt-Schleife zu bilden, die den entsprechenden Kondensator (C) auflädt, so dass der entsprechende Vorspannungsstrom (I_{bias}) derart ist, dass an dem entsprechenden Eingangsknoten (N_{IN}) eine Spannung anliegt, die im Wesentlichen gleich der entsprechenden ersten Referenzspannung (V_{ref1}) ist;
und wobei die Rückkopplungsstufe (30) einen ersten Eingangsanschluss, der so konfiguriert ist, dass er auf eine zweite Referenzspannung (V_{ref2}) eingestellt wird, einen zweiten Eingangsanschluss und einen jeweiligen Ausgangsanschluss aufweist, wobei die Rückkopplungsstufe (30) ferner einen Rückkopplungsverstärker (32) umfasst, dessen Ausgangsanschluss mit dem Ausgangsanschluss der Rückkopplungsstufe (30) gekoppelt ist; und wobei jede Pixelschaltung (10) ferner einen jeweiligen ersten Schalter (S₁), der so steuerbar ist, dass er den entsprechenden Kondensator (C) mit/von dem Ausgangsanschluss der Rückkopplungsstufe (30) koppelt/entkoppelt, und einen jeweiligen zweiten Schalter (S₂) umfasst, der so steuerbar ist, dass er den Ausgangsanschluss des entsprechenden Verstärkers (20) mit/von dem zweiten Eingangsanschluss der Rückkopplungsstufe (30) koppelt/entkoppelt;
wobei die integrierte elektronische Schaltung **dadurch gekennzeichnet ist, dass** die Rückkopplungsstufe (30) elektrisch steuerbar ist, um alternativ in einer ersten Konfiguration, in der ein erster und ein zweiter Eingangsanschluss des Rückkopplungsverstärkers (32) jeweils mit dem ersten und dem zweiten Eingangsanschluss der Rückkopplungsstufe (30) gekoppelt sind, und in einer zweiten Konfiguration, in der der erste und der zweite Eingangsanschluss des Rückkopplungsverstärkers (32) jeweils mit dem Ausgangsanschluss des Rückkopplungsverstärkers (32) und mit dem ersten Eingangsanschluss der Rückkopplungsstufe (30) gekoppelt sind, derart zu arbeiten, dass der Rückkopplungsverstärker (32) als Spannungsfolger arbeitet und die zweite Referenzspannung (V_{ref2}) an den Ausgangsanschluss der Rückkopplungsstufe (30) überträgt.

2. Integrierte elektronische Schaltung (10, 12) nach Anspruch 1, wobei jede Pixelschaltung (10) derart konfiguriert ist, dass der jeweilige zweite Transistor (M₂) von demselben Vorspannungsstrom (I_{bias}) durchflossen wird, der von dem entsprechenden ersten Transistor (M₁) injiziert wird.

3. Integrierte elektronische Schaltung (10, 12) nach Anspruch 1 oder 2, wobei der Rückkopplungsverstärker (32) ein Transkonduktanzverstärker ist.

4. Integrierte elektronische Schaltung (10, 12) nach einem der vorhergehenden Ansprüche, ferner umfassend eine Steuerschaltungsanordnung (12), die konfiguriert ist, um den ersten und zweiten Schalter (S₁, S₂) der Pixelschaltungen (10) zu steuern, um die Rückkopplungsstufe (30) mit jeweils einer Pixelschaltung (10) zu koppeln, wobei die Steuerschaltungsanordnung (12) ferner konfiguriert ist, um die Rückkopplungsstufe (30) so zu steuern, dass, wenn die Rückkopplungsstufe (30) mit einer Pixelschaltung (10) gekoppelt ist, die Rückkopplungsstufe (30) in der ersten Konfiguration arbeitet; wobei die Steuerschaltungsanordnung (12) ferner konfiguriert ist, um erste Zeiträume (25), in denen die Pixelschaltungen (10) einzeln nacheinander mit der Rückkopplungsstufe (30) gekoppelt sind, mit zweiten Zeiträumen (27) zu wechseln, wobei die Rückkopplungsstufe (30) von den Pixelschaltungen (10) entkoppelt ist und in der zweiten Konfiguration arbeitet.

5. Integrierte elektronische Schaltung (10, 12) nach einem der vorhergehenden Ansprüche, wobei der zweite Transistor (M₂) jeder Pixelschaltung (10) einen jeweiligen zweiten Leitungsanschluss aufweist, der auf ein erstes Referenzpotential (GND) eingestellt ist; und wobei der erste Transistor (M₁) jeder Pixelschaltung (10) einen jeweiligen zweiten Leitungsanschluss aufweist, der auf ein zweites Referenzpotential (VDD) eingestellt ist; und wobei der Kondensator (C) jeder Pixelschaltung (10) einen jeweiligen ersten Anschluss, der auf das erste oder das zweite Referenzpotential (VDD) eingestellt ist, und einen jeweiligen zweiten Anschluss aufweist, der mit dem Steueranschluss des entsprechenden ersten Transistors (M₁) gekoppelt ist.

6. Integrierte elektronische Schaltung (10, 12) nach einem der vorhergehenden Ansprüche, wobei der erste und der zweite Transistor (M₁, M₂) jeder Pixelschaltung (10) P-Kanal-Anreicherungs-MOSFET-Transistoren sind.

7. Integrierte elektronische Schaltung (10, 12) nach einem der vorhergehenden Ansprüche, ferner umfassend eine Vorspannungsschaltungsanordnung (12), die mit den ersten Eingangsanschlüssen der Verstärker (20) der Pixelschaltungen (10) gekoppelt ist und konfiguriert ist, um an jedem der Eingangsanschlüsse eine entsprechende erste Referenzspannung (V_{ref1}) einzustellen.

8. Integrierte elektronische Schaltung (10, 12) nach einem der vorhergehenden Ansprüche, wobei der Verstärker (20) jeder Pixelschaltung (10) konfiguriert ist, um ein Ausgangssignal (V_{OUT}) an seinem eigenen Ausgangsanschluss zu erzeugen; wobei die integrierte elektronische Schaltung (10, 12) ferner eine Leseschaltung (12) umfasst, die elektrisch steuerbar mit den Pixelschaltungen (10) koppelbar ist, um die jeweiligen Ausgangssignale (V_{OUT}) zu lesen.

9. Integrierte elektronische Schaltung (10, 12) nach einem der vorhergehenden Ansprüche, wobei der Verstärker (20) jeder Pixelschaltung (10) ein Transkonduktanzverstärker ist.

## Revendications

1. Circuit électronique intégré (10, 12) pour un module de sonde implantable (11), comprenant un certain nombre de circuits de pixels (10) comprenant chacun :
- une électrode (15) configurée pour entrer en contact avec un tissu biologique ;
- un étage de polarisation (M₁, C) comprenant un condensateur (C) et un premier transistor (M₁) ayant une borne de commande respective et une première borne de conduction respective, qui sont respectivement couplées au condensateur (C) et à un nœud d'entrée (N_{IN}), le premier transistor (M₁) étant configuré pour injecter dans le nœud d'entrée (N_{IN}) un courant de polarisation (I_{bias}) qui dépend de la charge du condensateur (C) ;
- un second transistor (M₂), qui a une borne de commande respective et une première borne de conduction respective, qui sont respectivement couplées à l'électrode (15) et au nœud d'entrée (N_{IN}) ;
- un amplificateur (20) ayant une première borne d'entrée, configurée pour être réglée sur une première tension de référence (V_{ref1}) correspondante, et une seconde borne d'entrée, qui est couplée au nœud d'entrée (N_{IN}) ;
ledit circuit électronique intégré (10, 12) comprenant en outre :
- un étage de rétroaction (30) pouvant être commandé électriquement de manière à être alternativement couplé ou découplé de l'étage de polarisation (M₁, C) et de l'amplificateur (20) de chaque circuit de pixels (10), ledit étage de rétroaction (30) étant configuré pour former, lorsqu'il est couplé à l'étage de polarisation (M₁, C) et à l'amplificateur (20) d'un circuit de pixels (10), une boucle de mise à zéro automatique qui charge le condensateur (C) correspondant de sorte que le courant de polarisation (I_{bias}) correspondant soit tel que le nœud d'entrée (N_{IN}) correspondant présente une tension sensiblement égale à la première tension de référence (V_{ref1}) correspondante ;
et dans lequel l'étage de rétroaction (30) a une première borne d'entrée, configurée pour être réglée sur une seconde tension de référence (V_{ref2}), une seconde borne d'entrée et une borne de sortie respective, ledit étage de rétroaction (30) comprenant en outre un amplificateur de rétroaction (32), dont la borne de sortie est couplée à la borne de sortie de l'étage de rétroaction (30) ; et dans lequel chaque circuit de pixels (10) comprend en outre un premier interrupteur (S₁) respectif, qui peut être commandé de manière à coupler/découpler le condensateur (C) correspondant à la borne de sortie de l'étage de rétroaction (30), et un second interrupteur (S₂) respectif, qui peut être commandé de manière à coupler/découpler la borne de sortie de l'amplificateur (20) correspondant à la seconde borne d'entrée de l'étage de rétroaction (30) ;
ledit circuit électronique intégré étant **caractérisé en ce que** l'étage de rétroaction (30) peut être commandé électriquement de manière à fonctionner alternativement dans une première configuration, dans laquelle une première et une seconde borne d'entrée de l'amplificateur de rétroaction (32) sont respectivement couplées à la première et à la seconde borne d'entrée de l'étage de rétroaction (30), et dans une seconde configuration, dans laquelle la première et la seconde borne d'entrée de l'amplificateur de rétroaction (32) sont respectivement couplées à la borne de sortie de l'amplificateur de rétroaction (32) et à la première borne d'entrée de l'étage de rétroaction (30), de telle sorte que l'amplificateur de rétroaction (32) fonctionne comme un suiveur de tension et transfère la seconde tension de référence (Vre_{f2}) à la borne de sortie de l'étage de rétroaction (30).

2. Circuit électronique intégré (10, 12) selon la revendication 1, dans lequel chaque circuit de pixels (10) est configuré de telle sorte que le second transistor (M₂) respectif est traversé par le même courant de polarisation (L_{bias}) injecté par le premier transistor (M₁) correspondant.

3. Circuit électronique intégré (10, 12) selon la revendication 1 ou 2, dans lequel l'amplificateur de rétroaction (32) est un amplificateur de transconductance.

4. Circuit électronique intégré (10, 12) selon l'une quelconque des revendications précédentes, comprenant en outre un circuit de commande (12) configuré pour commander les premier et second interrupteurs (S₁,S₂) des circuits de pixels (10) de manière à coupler l'étage de rétroaction (30) à un circuit de pixels (10) à la fois, ledit circuit de commande (12) étant en outre configuré pour commander l'étage de rétroaction (30) de manière à ce que, lorsque ledit étage de rétroaction (30) est couplé à un circuit de pixels (10), l'étage de rétroaction (30) fonctionne dans la première configuration ; ledit circuit de commande (12) étant en outre configuré pour alterner les premières périodes de temps (25), au cours desquelles les circuits de pixels (10) sont individuellement couplés, en succession, à l'étage de rétroaction (30), avec les secondes périodes de temps (27), au cours desquelles l'étage de rétroaction (30) est découplé des circuits de pixels (10) et fonctionne dans la seconde configuration.

5. Circuit électronique intégré (10, 12) selon l'une quelconque des revendications précédentes, dans lequel le second transistor (M₂) de chaque circuit de pixels (10) a une seconde borne de conduction respective, qui est réglée sur un premier potentiel de référence (GND) ; et dans lequel le premier transistor (M₁) de chaque circuit de pixels (10) a une seconde borne de conduction respective, qui est réglée sur un second potentiel de référence (VDD) ; et dans lequel le condensateur (C) de chaque circuit de pixels (10) a une première borne respective, réglée sur le premier ou le second potentiel de référence (VDD), et une seconde borne respective, couplée à la borne de commande du premier transistor (M₁) correspondant.

6. Circuit électronique intégré (10, 12) selon l'une quelconque des revendications précédentes, dans lequel les premier et second transistors (M₁,M₂) de chaque circuit de pixels (10) sont des transistors MOSFET à canal P à enrichissement.

7. Circuit électronique intégré (10, 12) selon l'une quelconque des revendications précédentes, comprenant en outre un circuit de polarisation (12) couplé aux premières bornes d'entrée des amplificateurs (20) des circuits de pixels (10) et configuré pour fixer, sur chacune desdites bornes d'entrée, une première tension de référence (V_{ref1}) correspondante.

8. Circuit électronique intégré (10, 12) selon l'une quelconque des revendications précédentes, dans lequel l'amplificateur (20) de chaque circuit de pixels (10) est configuré pour générer un signal de sortie (V_{OUT}) sur sa propre borne de sortie ; ledit circuit électronique intégré (10, 12) comprenant en outre un circuit de lecture (12) pouvant être couplé de manière électriquement commandable aux circuits de pixels (10), de façon à lire les signaux de sortie respectifs (V_{OUT}).

9. Circuit électronique intégré (10, 12) selon l'une quelconque des revendications précédentes, dans lequel l'amplificateur (20) de chaque circuit de pixels (10) est un amplificateur de transconductance.
